Europäisches Patentamt

European Patent Office

Office européen des brevets

(11) Publication number: **0 072 205**
**B1**

(12) **EUROPEAN PATENT SPECIFICATION**

(45) Date of publication of patent specification: **30.10.85**

(21) Application number: **82304134.8**

(22) Date of filing: **05.08.82**

(51) Int. Cl.⁴: **A 61 B 1/00,** A 61 B 1/06,
G 02 B 23/16

(54) **Endoscope cover glass fitting.**

(30) Priority: **05.08.81 JP 123494/81**

(43) Date of publication of application:
**16.02.83 Bulletin 83/07**

(45) Publication of the grant of the patent:
**30.10.85 Bulletin 85/44**

(84) Designated Contracting States:
**AT BE CH DE FR GB IT LI NL SE**

(56) References cited:
**DE-U-6 913 243**
**US-A-2 466 365**
**US-A-4 222 375**

(73) Proprietor: **OLYMPUS OPTICAL CO., LTD.**
**43-2, 2-chome, Hatagaya Shibuya-ku**
**Tokyo 151 (JP)**

(72) Inventor: **Hashiguchi, Toshihiko**
**8-3-15 Seishin Sagamihara-shi**
**Kanagawa-ken (JP)**

(74) Representative: **Kennedy, Victor Kenneth et al**
**G.F. REDFERN & CO. Marlborough Lodge 14**
**Farncombe Road**
**Worthing West Sussex BN11 2BT (GB)**

Courier Press, Leamington Spa, England.

EP 0 072 205 B1

## Description

This invention relates to an endoscope cover glass fitting for mounting a cover glass to seal the end of an insertion sheath containing an observing optical system of an endoscope.

An endoscope is an instrument provided with an observing optical system whereby an image is formed of an object in a place difficult to directly observe by an objective optical system arranged in the elongate insertion sheath, which is inserted to position its tip adjacent the desired object, which can then be observed from the rear of the instrument via an eyepiece mounted on a grip portion, being transmitted by an optical relay system, and such instruments have recently become extensively used in the industrial and medical fields.

Such endoscopes may be soft endoscopes, wherein the insertion sheath is soft and pliant, or hard endoscopes, wherein the insertion sheath is rigid and substantially linear.

In each of these endoscopes, the window aperture at the tip of the observing optical system is sealed, either by a transparent cover glass separately provided, or possibly by a cover glass formed by a surface of a lens of the system. However, in conventional endoscopes there have been problems, in that the cover glass lies at the bottom of a recess formed by the rim of retaining member that is arranged to press and support the cover glass, and therefore dust and liquid will be likely to accumulate in the recessed part and impair observation, and when a deposit of dust and/or liquid has formed, it is difficult to wipe off.

A further problem is that peripheral internal rim of the retaining member, projecting as it does from the cover glass, in the tip of the insertion sheath, may hurt the wall surface of an internal organ or the like when the instrument is inserted into a body cavity.

One known endoscope seeks to overcome this latter problem by forming the peripheral internal rim of the retaining member with a tapered or chamfered surface facing forward, so as to provide a ramp down to the cover glass surface.

In this known example, there have been problems, in that the residual thickness of the part of the tapered chamfered retaining member projecting from the cover glass surface becomes too thin to permit working precision, and there is a danger that serrated notches will be formed at the inner boundary part that lies in contact with the cover glass surface, which enables dust or the like to accumulate in the concavities or valleys of the notches, may impair observation, and, if the amount ground away during chamfering is larger than a predetermined amount, even within precise tolerance working the function of retaining and supporting pressure against the cover glass will be insufficient.

Thus, in the last-described known structure, it has been very hard to obtain an optimum degree of chamfering with working precision.

One object of the present invention is to provide an endoscope cover glass fitting which is easy to work, presents no problems regarding the working precision and can positively fix the cover glass, whilst making it hard for dust and liquid to accumulate to obstruct the observation, and ensure that even if they are deposited they will be readily removed, and ensuring there is little danger of any internal organ or the like being hurt when an endoscope is inserted or moved within a body cavity.

According to one exemplary embodiment there is provided an endoscope cover glass fitting for an endoscope wherein a cover glass is contained and fixed by an annular retaining member substantially fitting in the end of an inner tube containing an observing optical system by which the image of an object is formed and transmitted to be observed from the rear via an eyepiece on an operating grip portion, characterised in that the outer periphery of the outer end face of the cover glass is incised to form a tapered edge surface and a mating retaining member having an inwardly projecting rim contacting said incised surface is provided which surrounds at least part of the peripheral edge of said cover glass.

The invention will now be described with reference to the drawings in which:—

Figure 1 is a side view showing a known hard endoscope;

Figure 2 is a side view showing details of the tip of the insertion sheath with a retaining member that is not chamfered, as used in the known construction shown in Figure 1;

Figure 3 is a magnified sectional view of details of another known construction in which the tip of the insertion sheath has a retaining member that is chamfered;

Figure 4 is a sectional view showing essential details of the sealed tip in the first exemplary embodiment of the present invention;

Figures 5a and 5b are sectional views showing a first step of fixing a cover glass receiving seat for the embodiment shown in Figure 4;

Figures 6a and 6b are sectional views showing a step of forming a light guide for the embodiment shown in Figure 4;

Figures 7a and 7b are sectional views showing a step of fitting and fixing a cover glass for the embodiment shown in Figure 4;

Figure 8 is a detailed sectional view showing the tip of a second exemplary embodiment of the present invention;

Figures 9a and 9b are sectional views showing details of a third exemplary embodiment of the present invention and a variant form thereof; and

Figure 10 is a sectional view showing details of a fourth exemplary embodiment of the present invention.

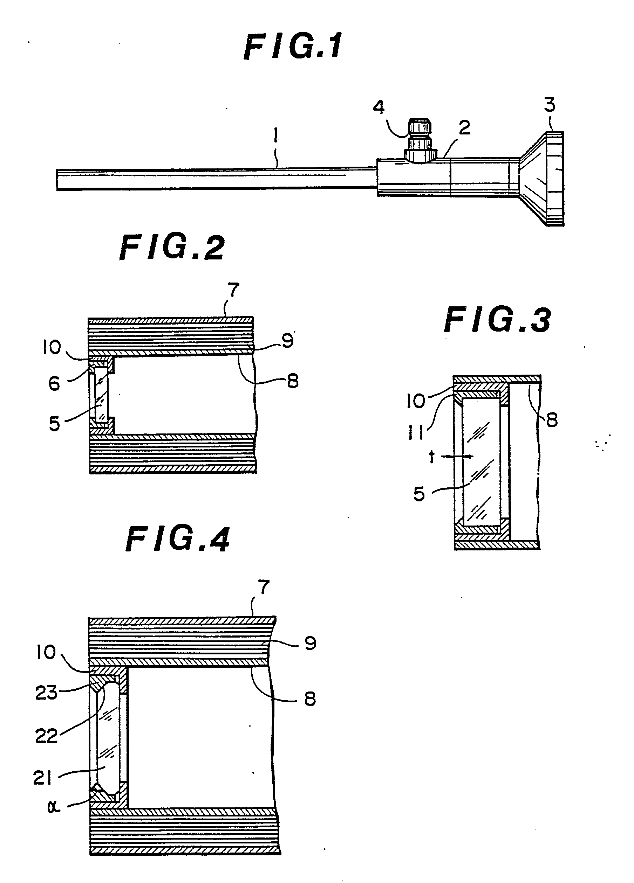

Prior to explaining embodiments of the present invention, the known examples of endoscope cover glass fittings will be described with reference to Figures 1 to 3.

A hard endoscope is formed of rigid insertion sheath 1 to be guided into a body cavity, for example, by a trocar or like aid, being mounted on an operating grip portion 2 having an eyepice objective 3 and a coupler 4 for connecting a light guide cable.

As shown in section in Figure 2, the tip of the insertion sheath 1 is formed by forming an annular retaining member 6 for a cover glass 5. As shown in Figure 2, an inner tube 8 containing an observing optical system is contained in an outer tube 7 forming the outer wall of the insertion sheath 1 and fibres of an illuminating light guide 9 are arranged between the inner tube 8 and outer tube 7. An observing objective system (not illustrated) is contained in the inner tube 8. The cover glass 5 is fitted and fixed to the tip of the inner tube 8 by the retaining member 6. A cover glass receiving seat 10 is arranged between the outer peripheral surface of the retaining member 6 and the internal peripheral surface of the inner tube 8, to securely fix the cover glass 5.

The retaining member 6, the opening at the tip of the section is not tapered, as shown in Figure 2, so that water and dust could accumulate in the recess at this face, and, in cases where the instrument is to be inserted into a body cavity, unnecessary substances will tend to accumulate in the recess and will be hard to wipe off.

Also, when this tip is inserted into a body cavity, it will be likely to hurt an internal organ or the like within the human body.

Therefore, in another known example, shown in Figure 3, the retaining member 6 is shamfered to taper the internal wall on the outside to open widely from the glass 5 so as to form a safer retaining member 11. However, in this case, the thickness $t$ of the tapered retaining member 11 projecting from the front surface of the cover glass 5 formed after the chamfering will become too thin from the viewpoint of working precision, and the acute angle at the internal rim part in contact with the cover glass will tend to produce serrated notches and, if the amount ground in chamfering exceeds a predetermined level, the function of retaining the cover glass 5 will not be effectively maintained.

Thus, optimum chamfering has been very difficult to achieve with working precision.

The present invention is intended to avoid the problems of the above-mentioned conventional known examples.

Respective embodiments of endoscope cover glass fittings constructed in accordance with the present invention will now be described with reference to the remaining Figures.

The first exemplary embodiment, shown in Figure 4, the known cover glass 5 of the conventional known constructions shown in Figures 2 and 3, and also the retaining member 11 shown in Figure 3 are replaced by elements formed as follows.

The periphery of one face of the cover glass 21 is cut at an angle which may be freely selected, and in this example is of 45 degrees, to provide an incised surface 22. The face provided with this incised surface 22 is arranged as the entrance end face, which may be an observing optical element end face or merely a front face of a plane cover glass, and in this embodiment a small incision is provided on the periphery of the other face (this small incision is not essential, and an be omitted in modifications of this and the later-described embodiments).

A retaining member 23 shaped to contact the incised surface 22 and the outer periphery of the cover glass 21, having an internal rim forming an apex at the outer periphery of the entrance end face of the cover glass 21, and having a forwardly tapered surface opening at a wide angle, is fixed in the cover glass receiving seat 10 by press-fitting or by use of a bonding agent, between the cover glass 21 and the seat 10.

As illustrated, this retaining member 23 projects toward the central axis of the inner tube 8 along the incised surface 22, from the outer periphery of the cover glass 21 and the cross-section of this inwardly projecting rim is shaped substantially like an isosceles triangle in this case, but this particular shape is not essential.

In the first exemplary embodiment, wherein the cover glass 21 is fitted and fixed in such a structure, when the entrance end face for the observing light (particularly as defined by its retaining member 23) is only ground down to the rim tip, an observing (optical) end face as illustrated will be formed, and can be reliably and easily produced in less steps than heretofore.

According to this embodiment, the rim tip of the retaining member 23, in contact with the cover glass 21, has an angle α between the face extending forward and the face in contact with the incised face 22, and is of such a comparatively large angle, about 90 degrees, that it can be easily worked without becoming deformed during the working operation. Further, even where the retaining member 23 becomes very thin, close to the front face of the cover glass 21, any serrated notches such as are formed in the conventional example described above, will not be likely to be made either during the working, or after the forming operation, and therefore the working precision will not be a problem and the functions of pressing and fixing the cover glass 21 will not be impaired.

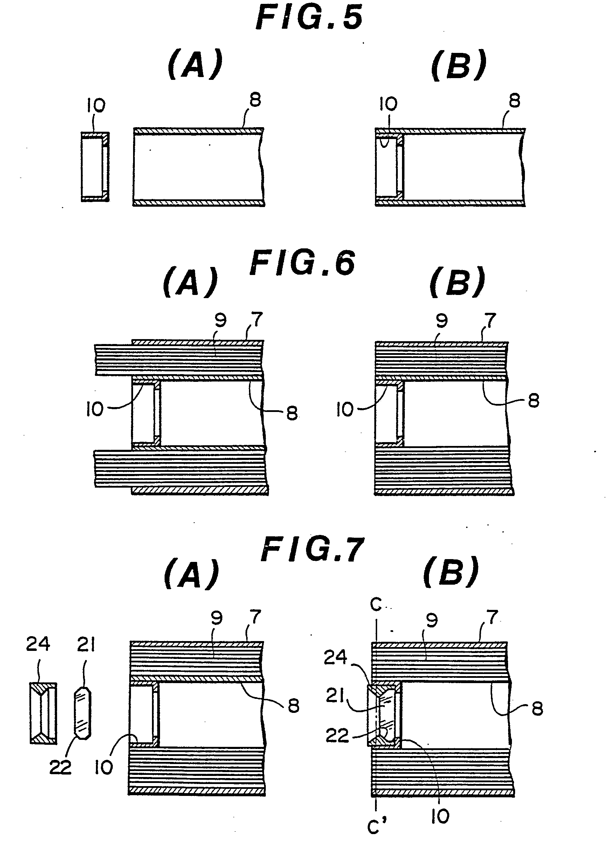

The steps of forming the above described first exemplary embodiment will be explained in the following with reference to Figures 5 to 7. A substantially annular cover glass receiving seat 10, in which an engaging peripheral flange part is provided for engaging the outer periphery of the inner face of the cover glass 21, as shown in Figure 5a, and is inserted into the end of the inner tube 8 that is contained in the outer tube 7 (not illustrated), and fixed as shown in Figure 5(b).

Then, the light guide 9 for transmitting an illuminating light is inserted to extend from a light input connector 4 (see Figure 1), extending between the outer tube 7 and inner tube 8 is shown in Figure 6(a), and is cut to be flush with the tips of the outer tube 7 and inner tube 8, as shown in Figure 6(b).

As shown in Figure 7(a), a cover glass 21 having an incised surface 22 is formed, and a retaining member 24 with an inwardly projecting rim which contacts the incised surface 22, and extends around the outer periphery of the cover glass 21, the part in contact with the incised surface 22 being substantially (isosceles) triangular in cross-section. These are assembled by press-fitting or fixed with a bonding agent in the cover glass receiving seat 10 that is already fixed within the inner tube 8, so that the cover glass 21 is securely located, as shown in Figure 7(b).

The thus fixed tip surface can then be ground down to the position shown by a line C—C′ in Figure 7(b). Thus, the cover glass 21 can be securely fitted and fixed by the retaining member 23 that projects by only a predetermined thickness from the front face of the cover glass 21.

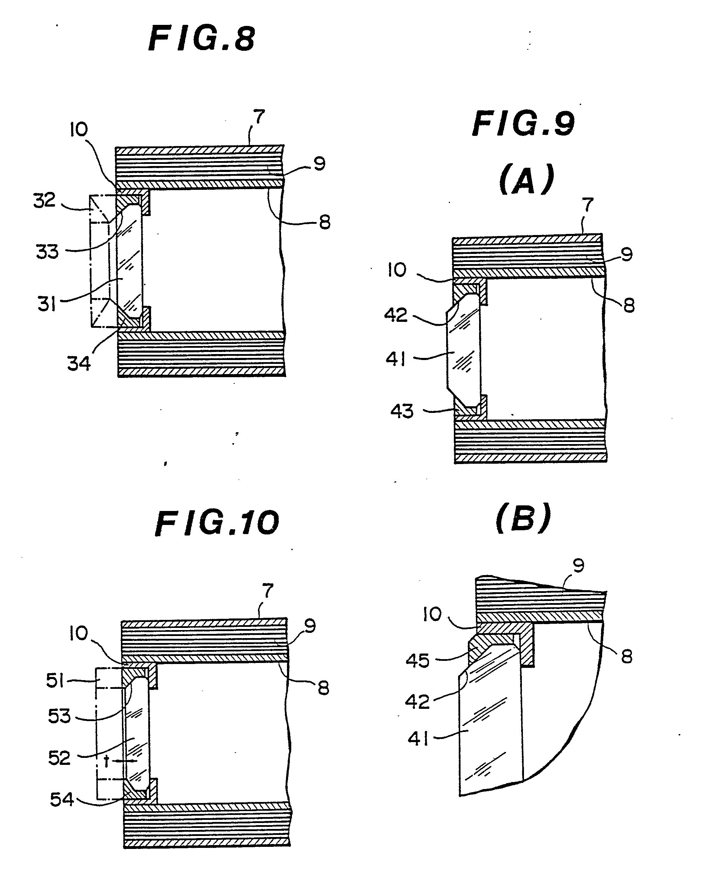

Figure 8 shows a second exemplary embodiment, wherein the observing optical end face and the front face of the cover glass 21 are in the same plane.

This embodiment has a cover glass 31 of substantially the same shape as that of the cover glass 21 with its incised surface 22 described with reference to the first embodiment, and a retaining member 32 may be of the same shape as described above (as is shown in Figure 8 by chain-dotted lines) or the incised surface 33 of the cover glass 31 may extend out as is shown by a broken line so as to make the retaining member 32 itself easy to make, by using a cover plate 31 of greater thickness.

When the substantially annular retaining member 32 is of different thickness on each side of the tapered rim tip, manufacture may be easier to make than in the former.

Further, in the first described embodiment, if the front edge of the incised surface 22 of the cover glass 21 does not coincide with the rim tip of the substantially triangular shape in view of working tolerances that may be allowed, then dust or the like will be likely to accumulate in this part, in the same manner as in the case of the known constructions. However, even in such case, in this embodiment, the dust or the like will be able to be removed after forming a retaining member 34 by grinding down as illustrated, and therefore there is a working advantage.

As the front face of the cover glass 31 is formed to be flush with the observing optical end face, there is no part in which water and dust can accumulate and cleaning will be easy.

The front face of the cover glass 31 is partly ground and removed, as illustrated, but need not always be ground and removed in a special operation, but may be initially ground to a position that will lie in the same plane as the ground end face of the light guide 9 or may be ground simultaneously with the grinding of the end face of the light guide 9. In either case the inwardly projecting rim 34 of the retaining member lies flush with the face of the plate 31 at the edge of the surface 33.

Figure 9(a) shows a third exemplary embodiment, wherein the front face of a cover glass 41 is formed to project out from the ground end face of the light guide 9 and other components.

In this embodiment as shown in Figure 9(a), as in the above described embodiments, an incised surface 42 is formed in the cover glass 41. In this embodiment, the retaining member 43 is initially formed to be substantially of the dimensions when completed. As shown in the magnified detail view in Figure 9(b), the retaining member 43 projects and its outer periphery is chamfered so as to avoid any possible damage to internal organs within a body cavity, when the endoscope is inserted, adjusted, or withdrawn.

In this embodiment, the cover glass 41 is fitted by being pressed or fixed with a bonding agent in the cover glass receiving seat 10 by pressure applied to the incised surface 42 and to the outer periphery by the retaining member 43, as described above. In such case, the outer periphery of the part projecting out of the ground end face of the light guide 9 is chamfered as mentioned above. In this case, the front face of the cover glass 41 may be ground before or after the retaining member 43 is fitted.

Even if the front face of the cover glass 41 partly projects as in this embodiment, the form of the incised surface 42 ensures that internal organs within the body cavity will not be damaged, and, as there is no recess, water and dust will not be likely to accumulate.

Figure 10 shows a fourth exemplary embodiment, wherein a retaining member 51 of a shape whose cross-section is different in radial thickness on both sides of the tapered surface, as shown by the chain-dotted lines in (Figure 10) is used instead of the retaining member 24 in the first embodiment described with reference to Figure 4 (or Figure 7). A retaining member 51 of such shape is easier to form, as described above.

In this case, the tapered width of the incised surface 53 of the cover glass 52 is set to be the same as or slightly larger than the tapered width of the retaining member in contact with it and, as illustrated, the projecting part of the retaining member 51 is ground away after the cover glass 52 is fixed, to form a finished member 54 slightly projecting forward of the front face of the cover glass 52.

The member 54 projects by the thickness $t$ from the front face of the cover glass 52, but this thickness $t$ is small enough to be substantially unlikely to accumulate dust and the like.

In the above described embodiments, means of fitting the cover glass to seal the opening in front of the objective, or seal the optical system at the tip of the insertion sheath are described. However, the present invention can be applied in the same manner for fitting a cover glass to seal the observing opening at the rear of the eyepiece.

The construction can be incorporated in a hard endoscope or a soft endoscope. The retaining member may be pre-formed or roughly formed *in situ* and finally shaped in a working operation.

## Claims

1. An endoscope cover glass fitting for an endoscope wherein a cover glass is contained and fixed by an annular retaining member substantially fitting in the end of an inner tube containing an observing optical system by which the image of an object is formed and transmitted to be observed from the rear via an eyepiece on an operating grip portion, characterised in that the outer periphery of the outer end face of the cover glass is incised to form a tapered edge surface and a mating retaining member having an inwardly projecting rim contacting said incised surface is provided which surrounds at least part of the peripheral edge of said cover glass.

2. An endoscope cover glass fitting according to Claim 1, characterised in that said retaining member has an outer face not smaller in diameter than the cover glass, but is slightly projected.

3. An endoscope cover glass fitting according to Claim 1, characterised in that the outer face of the cover glass is flush with the end face of the retaining member or projects slightly therefrom.

4. An endoscope cover glass fitting according to Claim 1, characterised in that the inner face of said cover glass is incised around its periphery to provide a tapered edge smaller than that on the outer face.

5. An endoscope having an insertion sheath containing an observing optical system sealed by an endoscope cover glass fitting, characterised in that said fitting is constructed in accordance with any one of the preceding Claims.

## Revendications

1. Adaptation de verre de protection d'endoscope pour endoscope, dans lequel un verre de protection est contenu et fixé par un élément de retenue annulaire s'adaptant exactement à l'extrémité d'un tube intérieur, contenant un système optique permettant de former et de transmettre l'image d'un objet pour l'observer de l'arrière au moyen d'un oculaire monté sur un élément de manoeuvre que l'on peut saisir à la main, adaptation caractérisée en ce que le pourtour extérieur de la face d'extrémité extérieure du verre de protection est taillé pour former une surface de bord allant en s'amincissant et en ce qu'on utilise un élément de retenue correspondant, muni d'un rebord en saillie intérieure venant en contact avec la surface taillée et entourant une partie au moins du bord périphérique de ce verre de protection.

2. Adaptation de verre de protection d'endoscope selon la revendication 1, caractérisée en ce que l'élément de retenue présente une face extérieure dont le diamètre n'est pas inférieure à celui du verre de protection, mais fait légèrement saillie par rapport à celui-ci.

3. Adaptation de verre de protection d'endoscope selon la revendication 1, caractérisée en ce que la face extérieure du verre de protection arrive à ras de la face d'extrémité de l'élément de retenue, ou fait légèrement saillie par rapport à celui-ci.

4. Adaptation de verre de protection d'endoscope selon la revendication 1, caractérisée en ce que la face intérieure du verre de protection est taillée sur son pourtour pour former un bord allant en s'amincissant, plus petit que celui de la face extérieure.

5. Endoscope muni d'une gaine d'insertion contenant un système optique d'observation scellé par une adaptation de verre de protection d'endoscope, endoscope caractérisé en ce que cette adaptation est réalisée selon l'une quelconque des revendications précédentes.

## Patentansprüche

1. Eine Endoskopdeckgläschenfassung für ein Endoskop, in der ein Deckgläschen enthalten und durch ein ringförmiges Halteglied befestigt ist, das im wesentlichen in das Ende eines Innenrohres passt, welches eine Beobachtungsoptik enthält, durch die das Bild eines Gegenstandes gebildet und derart übertragen wird, daß es von hinten durch ein Okular an einem Betätigungsgriffstück beobachtet werden kann, dadurch gekennzeichnet, daß der Außenumfang der äußeren Stirnfläche des Deckgläschens derart eingeschnitten ist, daß sich eine sich verjüngende Kantenfläche bildet und ein Gegenhalteglied vorgesehen ist mit einem nach innen ragenden Rand, der die genannte Einschnittsfläche berührt, welches wenigstens einen Teil der Umfangskante des gennanten Deickgläschens umgibt.

2. Eine Endoskopdeckgläschenfassung nach Anspruch 1, dadurch gekennzeichnet, daß das genannte Halteglied eine Außenfläche besistzt, die im Durchmesser nicht kleiner als das Deckglaschen ist, jedoch etwas hervorsteht.

3. Eine Endoskopdeckgläschenfassung nach Anspruch 1, dadurch gekennzeichnet, daß die Außenfläche des Deckgläschens bündig mit der Stirnfläche des Haltegliedes abschließt oder etwas davon hervorsteht.

4. Eine Endoskopdeckgläschenfassung nach Anspruch 1, dadurch gekennzeichnet, daß die Innenfläche des genannten Deckgläschens um ihren Umfang herum derart eingeschnitten ist, daß sich eine sich verjüngende Kante, die kleiner als die an der Außenfläche ist, bildet.

5. Ein Endoskop mit einer Einsatzhülle, welche eine durch eine Endoskopdeckgläschenfassung abgeschlossene Beobachtungsoptik enthält, dadurch gekennzeichnet, daß die genannte Fassung gemäß einem der vorhergehenden Ansprüche ausgebildet ist.

0 072 205

# FIG.1

# FIG.2

# FIG.3

# FIG.4

1

# FIG.5

**(A)**        **(B)**

10   8      10   8

# FIG.6

**(A)**        **(B)**

9   7      9   7

10    8      10    8

# FIG.7

**(A)**        **(B)**

24   21   9   7      C   9   7

24

21   8

22    8

22   10      C'   10

## FIG.8

## FIG.9
## (A)

## FIG.10

## (B)